# EUROPEAN PATENT SPECIFICATION

(11) **EP 0 682 507 B1**
(45) Date of publication and mention of the grant of the patent: **07.06.2000**
(21) Application number: 93920472.3
(22) Date of filing: 31.08.1993
(51) Int. Cl.: A61F 2/32, A61F 2/34

(54) **ACETABULAR CUP BODY PROSTHESIS AND INSPECTION GRID**
PFANNE FÜR HÜFTGELENKPROTHESE UND PRÜFGITTER
PROTHESE ACETABULAIRE A CORPS CUPULIFORME ET GRILLE DE CONTROLE

(30) Priority: 31.08.1992 US 938421
(43) Date of publication of application: 22.11.1995
(73) Proprietor: SMITH & NEPHEW, INC., Memphis, Tennessee 38116 (US)
(72) Inventor: SCHRYVER, Jeff, Cordova, TN 38018 (US); SHEA, Jeff, Memphis, TN 38120 (US); RYAN, Dawn, Michelle, Memphis, TN 38120 (US)
(74) Representative: Harrison Goddard Foote
(86) International application number: PCT/US93/08295
(87) International publication number: WO 94/05234

(56) References cited:
- EP-A- 0 499 475
- US-A- 4 676 798
- US-A- 4 770 661
- US-A- 5 314 487
- BEITZ & KÜTTNER 'DUBBEL, TASCHENBUCH FÜR DEN MASCHINENBAU' 1983 , SPRINGER-VERLAG , BERLIN 4.2 OBERFLÄCHENMESSUNG * page 1263 - page 1264 *

## Description

### BACKGROUND OF THE INVENTION:

### 1. Field Of The Invention:

The present invention relates to acetabular prosthetic devices wherein the prosthesis has a cup or shell with an inner concave surface that has a mirror-like polished surface. The shiny polished inner, concave surface faces a cup liner (e.g. polymeric) so that relative motion between the liner and shell will generate minimal liner debris. The polished shell concave surface has a roughness of preferably less than 20µm (eight micro inches). The body or shell can include radially extending bores therethrough that can be used as drill guides by a surgeon after the acetabular cup or shell has been placed in the patient's acetabulum. Holes can be drilled surgically into the underlying bone tissue using the drill guide openings so that one or more pegs for improved anchoring can be placed into the bores and affixed rigidly to the acetabular cup using an interference or wedge fit. The prosthesis (including cup or shell and rigidly affixed pegs) is thus anchored into the underlying surgical openings. Reference is made to EP-A-0 499 475, which teaches such a device.

The present application aims to solve the technical problem of locating defects in the polished inner surface of the acetabular cup body.

### SUMMARY OF THE PRESENT INVENTION:

The present invention provides an improved arrangement of an acetabular cup prosthesis and an inspection grid as defined in claims 1 and 11.

A polymeric cup liner registers and affixes to the cup body at the concave surface portion.

The cup body concave surface has a polished mirror-like surface that faces the liner for retarding liner debris generation. The polished surface has a roughness of preferably less than 20µm (eight micro inches). This surface finish in its interior spherically shaped dome or concave portion is the contact interface between the metallic shell and the acetabular polyethylene or polymer liner. The surface finish has numerous advantages.

First, it provides a low friction and low abrasion surface for distributing the contact forces between the polyethylene liner (UHMWPE) and the shell. This reduces the abrasive generation of polyethylene debris resulting from motion between the liner and shell.

This motion may come from a variety of mechanisms which include Poisson volumetric distortion of the polyethylene resulting in localised expansion and contraction of the surface of the liner against the shell as a result of loading of the femoral head in the liner, and the micro-motion which occurs from forces from the femoral head pushing the liner within and around the confines of the shell interior.

The mirror finish allows the use of optical non-contact inspection of the interior of the shell surface for checking the geometric correctness of the shell. The use of non-contact optical inspection methods allow complete checking of whole two and three dimensional surfaces at one time. The usual method of optical three-dimensional inspection is to project a regular pattern of light onto the surface which is to be inspected. The resultant two dimensional projection of the scene may be used to give highly accurate total surface measurement. Distortions in the regular pattern indicate distortions in the part surface and indicate deviations from the desired part geometry. This method is ineffective on highly smooth surfaces since the projected light of the regular pattern bounces off the measurement target and no two dimensional mapping is possible. In this device idea the highly smooth surface (which is smooth due to the requirements of paragraph 1 above) is further polished to act as a reflective mirror. This surface mirror then is used as a lens to view a two-dimensional pattern such as a grid drawn on a white sheet of paper or a series of concentric rings. Distortions in the viewed image then are a result of distortions of the lens and hence the surface which is desired to be measured. It is thereby possible to inspect the highly smooth surface of the acetabular device due to this polishing.

The method of inspection may be both by trained human inspectors and by image analysis performed by capturing the reflected image by a video camera, digitising the image, and using computer analysis to measure the amount of deviation of the pattern from the allowed surface geometry tolerance.

The advantages of this smooth and polished surface is therefore to provide non-contact, and therefore non-destructive (non-scratching), measurement of the interior of an acetabular device. The inspection methods require a surface which allows a reflective resolution sufficient to provide adequate reflective image quality for analysis. Our current inspection limits require a surface finish of less then 20µm (eight micro-inches) to accomplish this quality of resolution.

The cup body according to independent claim 11 includes openings therethrough which can function as drill guides for the surgeon after the cup has been placed in the patient's acetabulum. The present invention affords improved fixation and stability of the component because pegs can be placed in the acetabular cup after it has been placed in position by the surgeon. The pegs can be easily installed from the concave side of the acetabular cup component notwithstanding the fact that the acetabular cup component has already been placed in operative position in the patient's acetabulum.

With the present invention, a multiplicity of pegs can be rigidly attached to the acetabular cup prosthesis body for the purpose of securing it in place in the acetabular bone. This can be done through an opening or bore which is interchangeably used for a desired peg.

With the present invention, the acetabular cup can be placed in it's desired position in the acetabulum by the surgeon. The pegs (as described more fully herein) are then added to the cup body and attached to the prosthesis in a rigid fashion. Each peg protrudes through the acetabular cup body and into the underlying bone tissue of the acetabulum to provide a mechanical locking of the acetabular cup (including pegs) into the pelvis. The surgeon can use a pre-drill before placing the peg or spike wherein the opening or bore in the acetabular cup body functions as a drill guide. Pegs can be selectively placed so that they are not aligned with each other but are at angles to each other which aids in the mechanical stability of the acetabular cup body.

The apparatus preferably uses a plurality of pegs that feature a taper or wedge lock, barb lock, or knurl lock, to form an interference fit, or compression friction lock, and a rigid connection with the acetabular cup at the drill guide openings. The interference fit assures a rigid connection between peg and cup body so that each peg and cup body move together, rather that relative to one another. Relative motion causes possible contact between a peg and any polymer liner, creating the problem of liner debris generation. The pegs are smooth along the distal portion thereof so that movement of the peg and cup as a unit will not disrupt adjacent bone tissue.

The present invention thus provides an improved acetabular cup prosthesis that includes an acetabular cup body or shell component, having an inner concave surface and an outer convex surface.

A plurality of openings extend between the inner and outer surfaces along radial lines that can merge substantially near a center of curvature of the inner concave surface of the cup body, the openings forming elongated bores surrounded by a bore wall portion of the acetabular cup body. The cup body or shell can be spherical in form, or not spherical in form (such as e.g., an egg-shaped cup or shell).

The plurality of openings are positioned to define drill guides so that during a surgical implantation of the prosthesis, the surgeon can selectively drill into the underlying tissue through one or more of the openings and form surgical openings therein in the underlying bone tissue.

There are preferably a plurality of peg members, each being insertable into and registering with one or more of the openings in the prosthesis body, the peg members having a first proximate end portion having means thereon for forming a rigid connection with the acetabular cup body at one of the openings and with the bore wall, and a second smooth distal end portion adapted to extend into the underlying tissue (e.g., into surgically formed openings) after the cup body has been implanted in a patient.

### BRIEF DESCRIPTION OF THE DRAWINGS:

For a further understanding of the nature and objects of the present invention, reference should be had to the following detailed description taken in conjunction with the accompanying drawings, in which like parts are given like reference numerals, and wherein:
FIGURE 1 is a side sectional view of a first embodiment of the apparatus of the present invention;
FIGURE 2 is a perspective view of the first embodiment of the apparatus of the present invention;
FIGURE 3 is an exploded perspective view of the first embodiment of the apparatus of the present invention;
FIGURE 4 - 4A are fragmentary views of the first embodiment of the apparatus of the present invention;
FIGURES 5A - 5C are bottom, side, and top views of peg portion of the first embodiment of the apparatus of the present invention;
FIGURES 6A - 6C are bottom, side, and top views of another peg as used with the first embodiment of the apparatus of the present invention;
FIGURES 7A - 7C are bottom, side, and top views of a spike member as used with the first embodiment of the apparatus of the present invention;
FIGURES 8, 9, 10, and 11 are peg members used with the first embodiment of the apparatus of the present invention including respectively thread lock, barb lock, taper lock, and knurl lock embodiments thereof;
FIGURES 12A - 12D are top views of locking pin members used with the taper locking embodiment of the spike;
FIGURE 13 is a perspective fragmentary view of the first embodiment of the apparatus of the present invention illustrating the peg locking screw portion thereof;
FIGURE 14 is a perspective fragmentary view illustrating an interface of the peg locking screw with one of the pegs of FIGURES 5A - 5C;
FIGURE 15 is a partial sectional view illustrating the peg of FIGURE 10;
FIGURE 16 is a perspective view of a second and preferred embodiment of the apparatus of the present invention;
FIGURE 17 is a fragmentary view illustrating a peg member used with the second embodiment of the apparatus of the present invention;
FIGURE 18 is a fragmentary view illustrating the closure member portion of the second embodiment of the apparatus of the present invention; and
FIGURE 19 is a fragmentary sectional view illustrating the second embodiment of the apparatus of the present invention;
FIGURE 20 is a another fragmentary sectional view illustrating the second embodiment of the apparatus of the present invention;
FIGURE 21 is a another fragmentary sectional view illustrating the cup portion of the second embodiment of the apparatus of the present invention;
FIGURE 22 is a fragmentary sectional view of the second embodiment of the apparatus of the present invention illustrating the cup and cup liner connection;
FIGURE 23 is a schematic diagram of the grid used to inspect the polished surface for distortions;
FIGURE 24 is a second embodiment of a test grid pattern used to inspect the highly polished surface portion of the acetabular cup prosthesis of the present invention;
FIGURE 25 is a schematic diagram of a test grid showing no surface defects; and
FIGURE 26 is schematic diagram showing local defects for a polished surface that has been inspected using the grid.

### DETAILED DESCRIPTION OF THE PREFERRED EMBODIMENT:

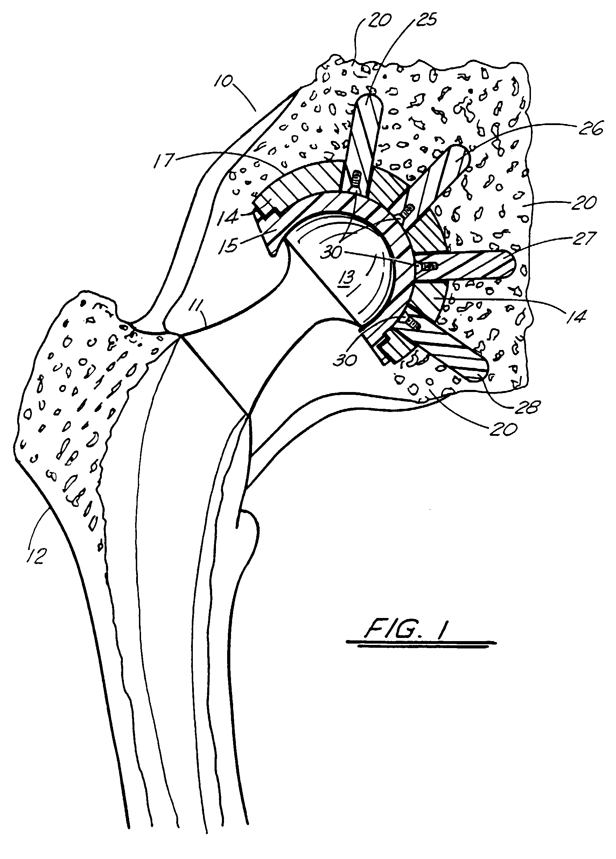

In Figure 1 there can be seen a sectional view of the first embodiment of the apparatus of the present invention designated generally by the numeral 10. In FIGURE 1, there can be seen a hip prosthesis member 11 mounted in a femur 12 of a patient. The hip prosthesis 11 includes an upper ball portion 13 that registers with the acetabular prosthetic apparatus 10 of the present invention.

The acetabular prosthesis 10 includes a cup or shell prosthesis body 14, preferably of a metallic material with a plastic liner 15 portion. The metallic cup body 14 includes an inner concave surface 16 and an outer convex surface 17. The surfaces 16, 17 are spaced apart, defining the thickness of the cup or shell 14. The cup body 14 provides a three-dimensional surface that is sintered to the outside surface 17 (such as sintered beads). The inside 17 is then machined after sintering. Another type of roughened outer surface 17 could be provided such as plasma sprayed metal, plasma sprayed hydroxyl apatite, or a mechanically textured or roughened surface. The shell or cup body 14 could have an exterior surface optimized for use with bone cement.

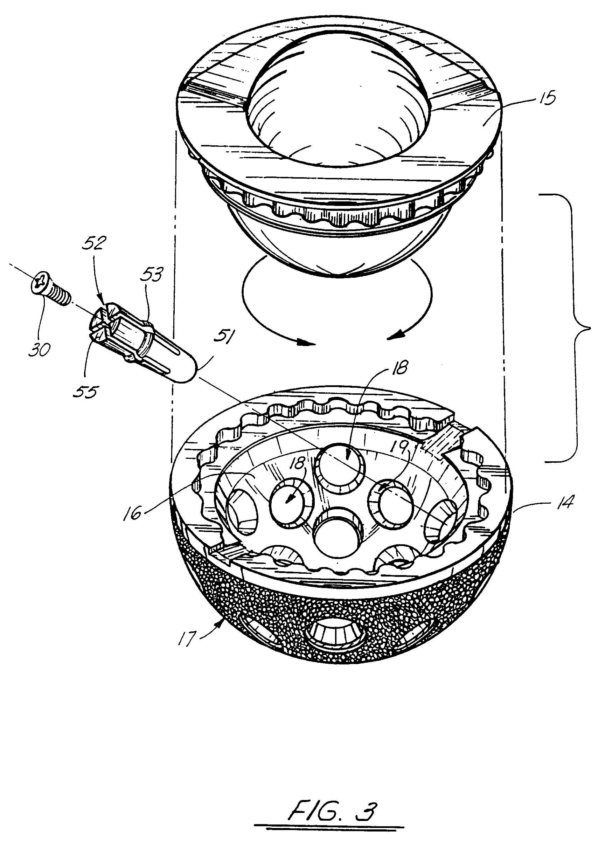

A plurality of openings 18 in form of preferably elongated bores extend between the inner concave surface 16 and the outer convex surface 17. These openings are in the form of bores having a bore wall 19 as seen in Figure 3. The openings 18 can function as drill guides for the surgeon. Therefore, once the metallic cup body 14 portion of the acetabular cup prosthesis 10 is placed in position in the patient's acetabulum as shown in Figure 1, the surgeon can simply drill through any one of the plurality of bores forming an opening in the underlying bone tissue designated generally be the numeral 20.

When the surgeon places the cup body 14 in the position shown in Figure 1, the plurality of bores 18 can act as a drill guide for the surgeon. The bore 19 walls of each opening 18 define a cylindrically shaped guide for a correspondingly sized drill. These openings allow the surgeon to form surgical openings in the underlying bone tissue 20.

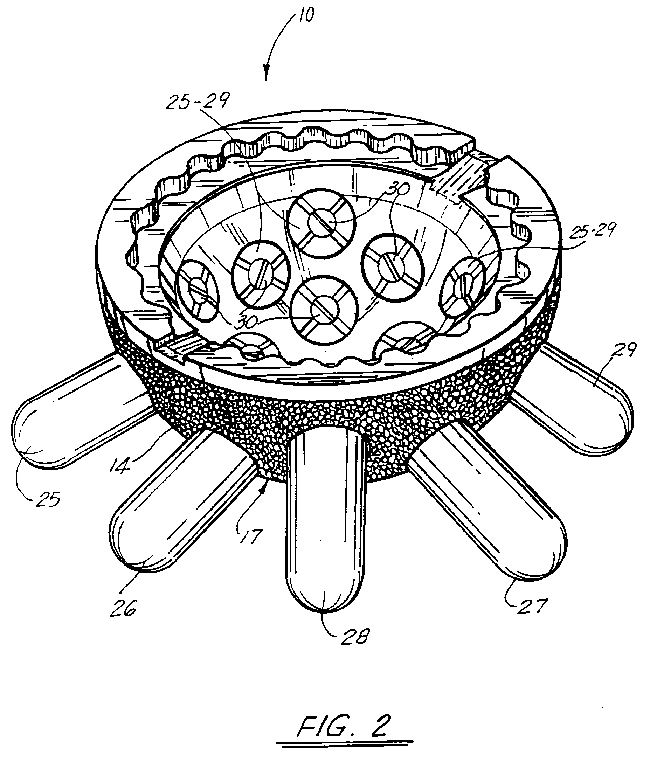

A selected surgically formed opening 18 is then occupied by a peg (and not necessarily each opening 18), such as one of the pegs 25 - 29, as seen in Figures 1 - 3 and 4A. In the preferred embodiment, each of the pegs 25 - 29 extends into the bone tissue at a different angular position with respect to the other pegs to provide a rigid anchor for the cup 14. Pegs 25-29 can be polymer, metal, or resorbable polymer.

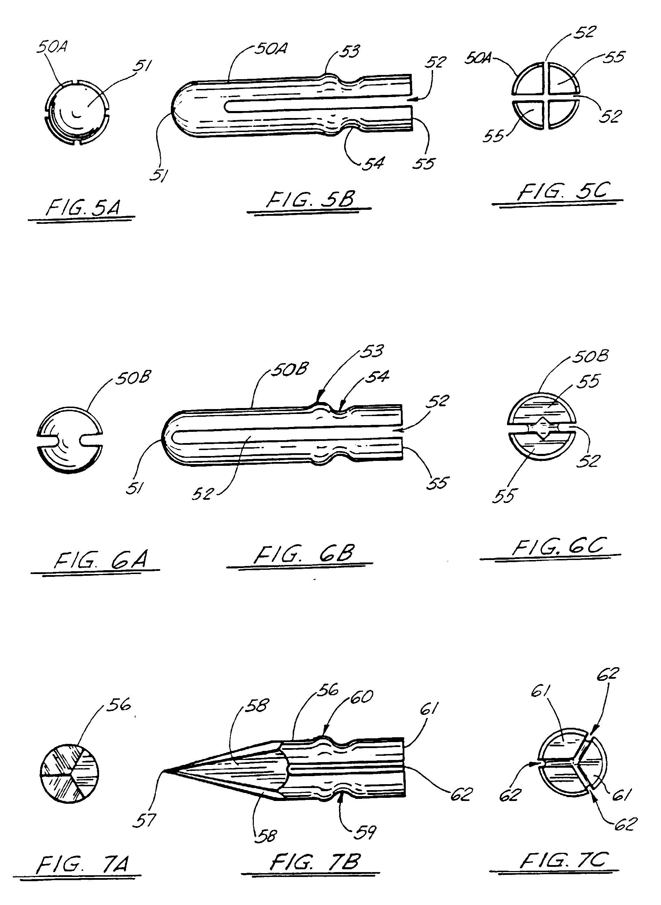

Once the pegs 25 - 28 are placed into operative position, a strong connection is formed between the outer surface of each peg 25 - 28 and the walls 19 of each opening or bore 18. In Figures 5A - 5C, 6A - 6C, 7A - 7C, and 8 - 11, various embodiments of the pegs, their respective attachments to the cup body 14 are illustrated.

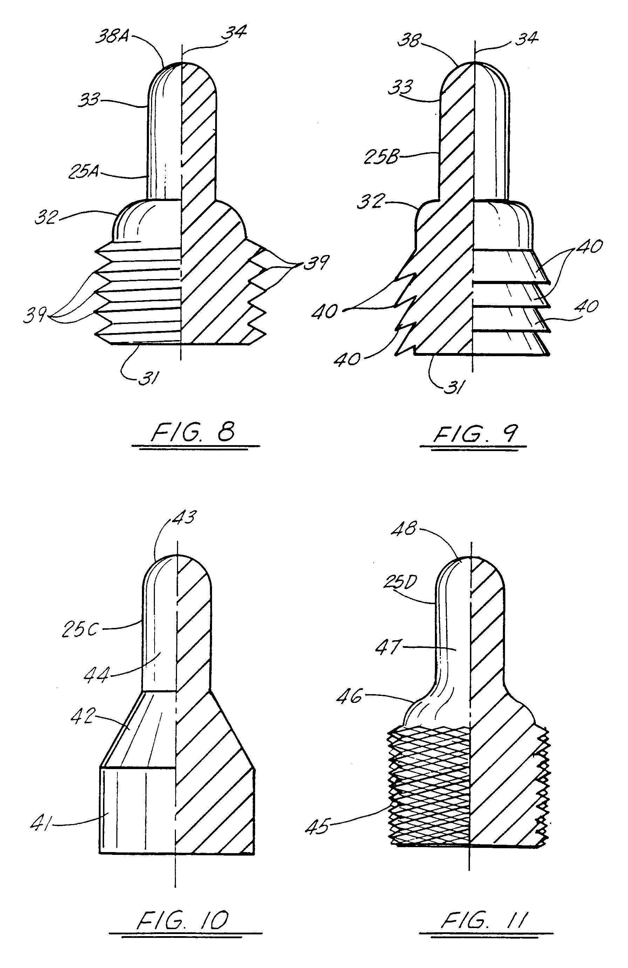

In Figure 8, peg 25A includes a proximate end portion 31 and a distal end portion 38A and a central longitudinal axis 34. A smaller diameter section 33 connects with a larger diameter section 32 that is covered with an external spiralling thread 39. The thread 39 bites into and interfaces with the cup body 14 at the wall 19 of each opening 18. The opening or bore 18 wall 19 can also be internally threaded to engage the thread 39.

In Figure 9, peg 25B is provided with a proximate end 31, a distal end 38, a smaller diameter section 33, and a larger diameter section 32 that carries a plurality of annular barb rings 40. When the peg 25B is forced into the opening 18, the barbs 40 form a tight fit with the cup body 14 at the wall 19 of each opening 18.

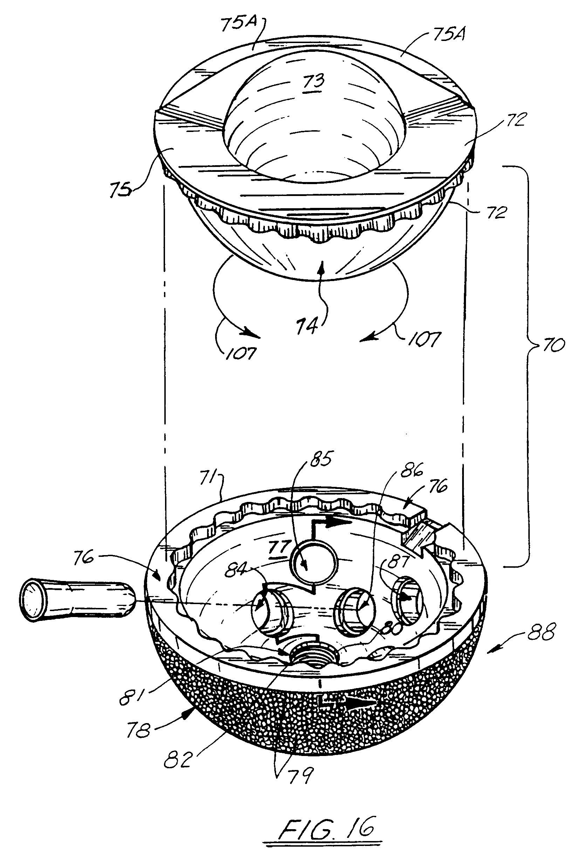

In the embodiment of Figures 10, 11, and 15, pegs 25C, 25D provide a proximate, larger diameter 41 end portion, a smaller generally cylindrical distal end portion 44, and a transitional frustro-conical section 42, and a curved end 43. Similarly, peg 25D has a proximate, larger diameter 45 portion, a curved annular transition section 46, a smaller and generally cylindrical section 47, and a curved end portion 48. The large diameter section 41 and the frustro-conical portion 42 can also be seen in Figure 15 in a partial sectional view. The larger diameter and frustro-conical sections 41, 42 surround an internally threaded bore 68 which accepts set screw 30. The screw 30 is sized to expand the enlarged 41 and frustro-conical 42 sections slightly when the peg 25C is placed in position within one of the bores 18 which would be similar in shape to the outside surface of frustro-conical portion 42 and enlarged portion 41.

When the set screw 30 is fully threaded into the threaded opening 68, so that the external thread 67 of the set screw 30 engages the internal thread 68 of the bore, a taper lock connection or interference fit is formed between the peg 25C and the wall 19 of opening 18. Internal threads 68 in Figure 15 can also be used as an extraction or holding means for placing and removing the peg 25c. Peg 25c would be used then without a screw 30.

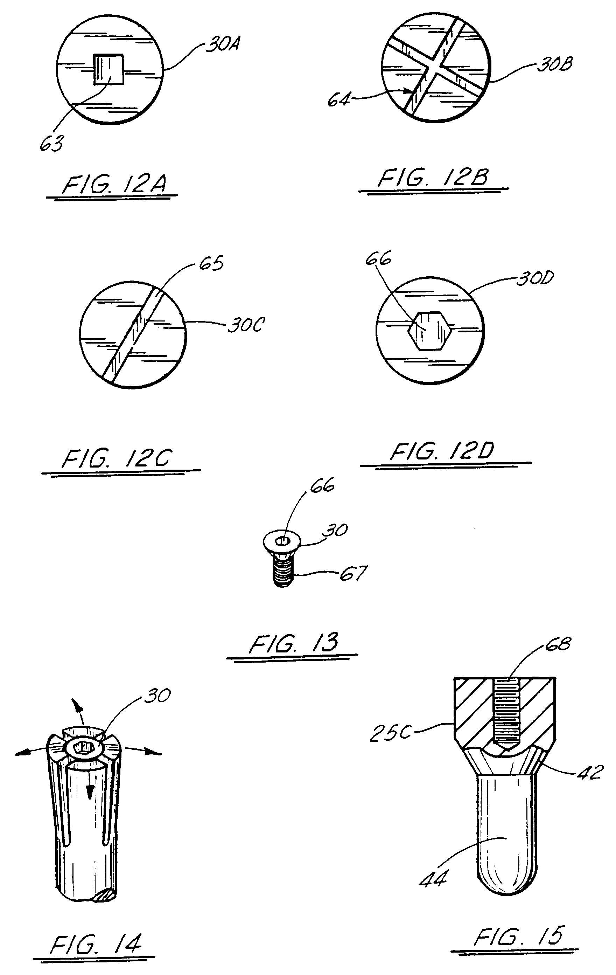

The set screw 30 can have different tooled sockets, as shown in Figures 12A - 12D. In Figure 12A, a set screw 30A includes a square tooled socket 63. In Figure 12B, the set screw 30B has a X-shaped slot 64 for receiving a Phillips-type screwdriver, for example. In Figure 12C, the set screw 30C has a single transverse slot 65 and in the embodiment of Figure 12B, the set screw 30B has an hexagonal tool socket 66. Other tooled sockets could be employed.

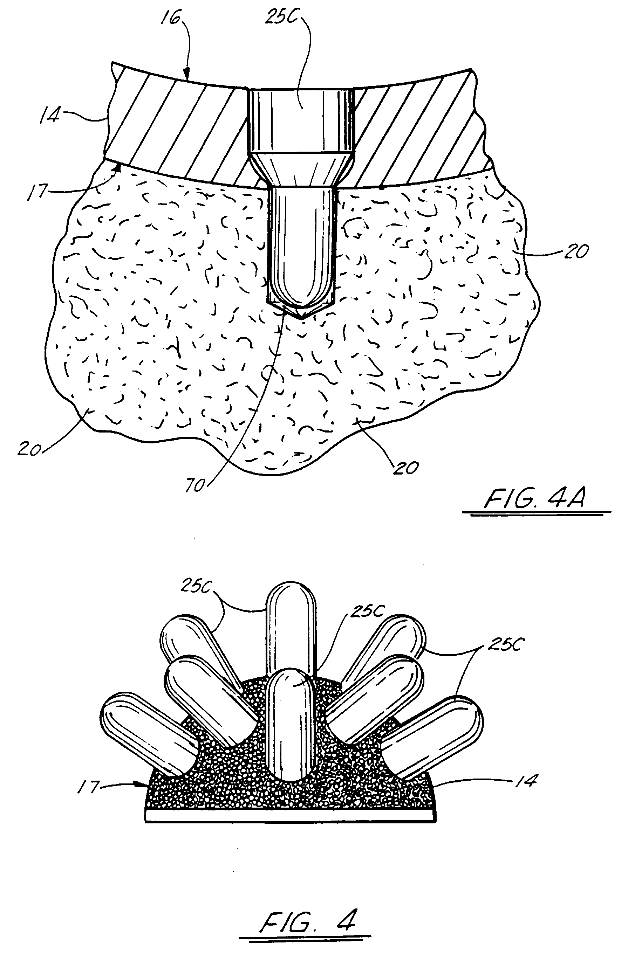

In Figures 4 - 4A, the pegs 25C can be shown extending from the convex 17 surface of cup body 14 and into a surgically formed opening 70 which is formed by the drill that penetrates the opening 18. In such a situation, the surgeon simply uses the opening 18 as a drill guide for a similarly shaped drill when forming surgical opening 70.

In Figures 5A - 5C, an alternate construction of the peg is illustrated, designated generally by the numeral 50A. Peg 50A includes a rounded or hemispherical distal end portion 51 and a proximate end portion 52 that includes a pair of longitudinally extending slots 49, forming four peg sections 55.

In the embodiments of Figures 6A - 6C, the peg 50B provides a hemispherical distal tip 51 and a single longitudinally extending transverse slot 49 forming two peg sections 55. Enlarged annular shoulder 53 and smaller diameter recess 54 are also provided in each of the embodiments of Figures 5A-5C and 6A - 6C.

In the embodiment of Figures 7A - 7C, a spike-shaped peg 56 is provided having a pointed tip portion 57, and a plurality of beveled surfaces 58 that connect with a cylindrical peg body portion that includes an enlarged annular section 60 and a smaller diameter constricted section 59. The proximate 61 end portion of the peg includes a longitudinally extending Y-shaped slot 62, as seen in Figures 7B and 7C.

Figures 16-22 illustrate a second and preferred embodiment of the apparatus of the present invention designated generally by the numeral 70. Acetabular cup apparatus 70 includes a cup body 71 to which can be removably affixed a plastic cup liner 72 made of polyethylene for example. Liner 72 has an inner concave surface 73 and an external convex surface 74. Liner 72 is in the form of a hemispherical member having an annular base 75 with a plurality of curved members extending around the periphery of liner 72 at base 75 and which interface with a similarly shaped circumferentially recess formed on cup body 71 to prevent rotation of liner 72 with respect to body 71. The cup body 71 is preferably metallic having an annular base 76 that defines a plane. The cup body 71 has an inner concave surface 77 and an external convex surface 78. The cup body 71 concave surface 77 is a shiny polished surface that faces the convex surface 74 of polymeric liner 72. The polished concave surface 77 has a roughness of less than 40µm (sixteen micro-inches) and preferably about 20µm (eight micro-inches). Such a highly polished surface 77 appears mirror-like. The polished concave surface 77 inhibits polymer liner debris generation.

External surface 78 can be covered with a plurality of small metallic beads or the like forming a bone ingrowth surface 79. The apex 80 of cup body 71 has a through opening 81 that has an internal thread 82 for attachment thereto of a tool for inserting and/or removing the cup body 71 form its position in the acetabular bone tissue of a patient.

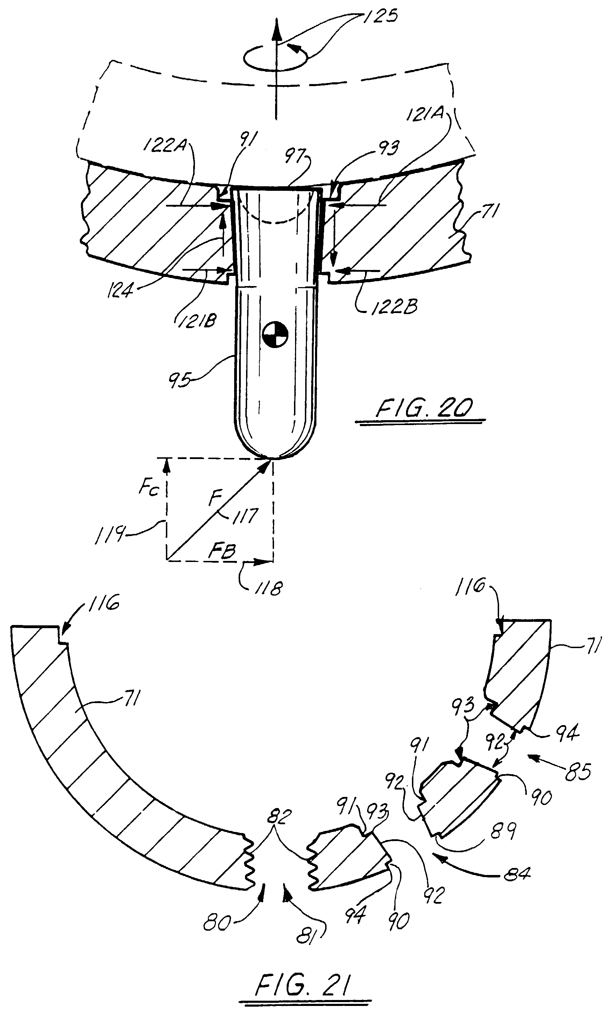

A plurality of four openings 84, 85, 86 and 87 are provided preferably in one quadrant 88 of cup body 71. Each opening 84-87 has an internal opening configuration that includes a larger diameter generally cylindrically shaped opening portion 91 and a smaller diameter opening portion 92 (Figure 20). The larger and smaller diameter sections 91, 92 are interfaced by an annular shoulder 93. The smaller diameter section 92 can be tapered from a point of maximum diameter adjacent annular shoulder 93 to a point of minimal diameter adjacent to the annular shoulder 89. A second larger diameter cylindrical section 90 meets outer edge 94 of each opening 84-87.

Pegs 95 can be selectively fitted into any one of the openings 84-87 during use. Each peg 95 has a tapered section 96 that includes a larger diameter circular base 97 defining a proximate end portion of peg 95. Distal end portion 98 of peg 95 is generally cylindrical and smooth, and includes a curved or hemispherical smooth tip 99.

In Figure 20 force arrows are used to demonstrate that the above-described connection between each peg 95 and the cup body 71 is a substantially rigid connection that produces load transfer between each peg member and the cup body of tension loads, compression loads, axial torsion loads, and bending moment loading. Thus, the peg 95 does not rotate nor back out with respect to the opening 85-87.

The force arrow 117 is an angled force having both a bending force component (Fb) designated as 118 and a compression force component (Fc) designated as 119. The force arrows 120 and 124 show shear forces at the interface between peg 95 and cup body 71 at a selected opening 84 - 87. The force arrows 121A,B are force arrows that are resisting the shear forces and the force arrows 122A,B are resisting bending forces.

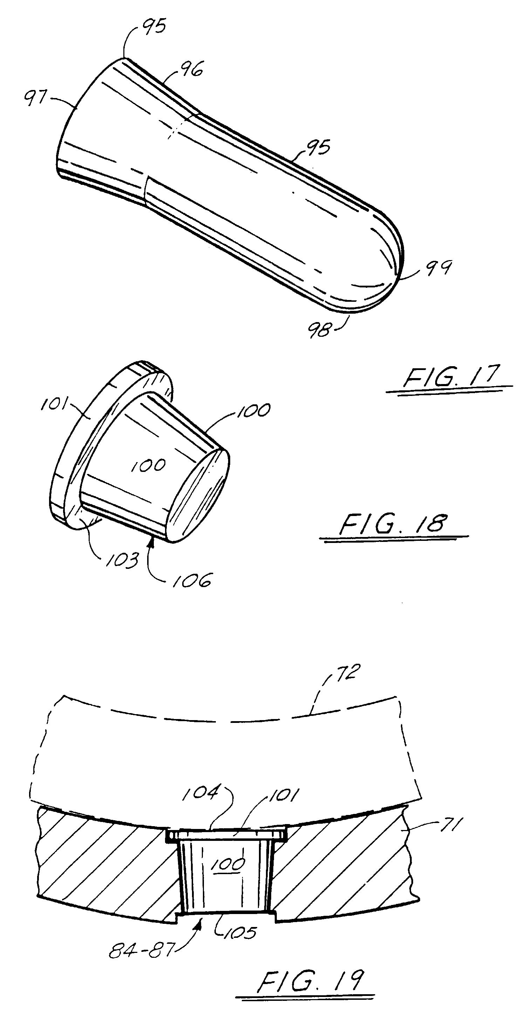

A closure member 100 is shown in Figures 18-19 and includes a larger section 101 and a smaller diameter section 102. Annular shoulder 103 forms an interface between the larger 101 and smaller 102 diameter sections. Closure member 101 has an enlarged circular base 104 defining its proximate end portion during use and a smaller circular flat distal end 105.

The larger diameter section 101 of closure member 100 can have a beveled annular wall 106 for forming a wedge type fit with the larger diameter section 91 of a particular selected opening 84-87. In this manner, the closure member 100 can be placed in any one of the selected openings 84-87 and pressed into the selected opening 84-87 by the user even after the cup body 71 has been placed into operative position. The user simply presses the closure member 100 into one of the selected openings 84-87 by accessing the cup body 71 from the concave 77 side.

The surgeon can close any one of the selected openings 84-87 using the closure member 100 after the cup body 71 has been placed in the desired operative position and anchored into position using one or more of the pegs 95. The closure member 100 may be placed in the shell 71 before implantation, such as during manufacturing, and then selectively removed by the surgeon before use of the shell 71. Each closure member 100 occupies a position in its selected opening 84-87 and between the convex 78 and concave 77 surfaces of shell or cup body 71.

Each of the openings 84-87 has a generally cylindrical smaller diameter section 92 that can act as a drill guide for the surgeon so that drilled openings can be made into the underlying bone tissue after the acetabular cup body 71 has been placed into position. This allows one or more pegs to be placed into one or more of the selected openings 84-87. Any of the selected openings 84-87 that are not selected can be plugged using the closure member 100. This prevents the flow of the polyethylene liner into any of the openings 84-87 that are not occupied by a peg 95. The closure member 100 friction fits into the above-mentioned larger diameter portion 91 of the openings 84-87.

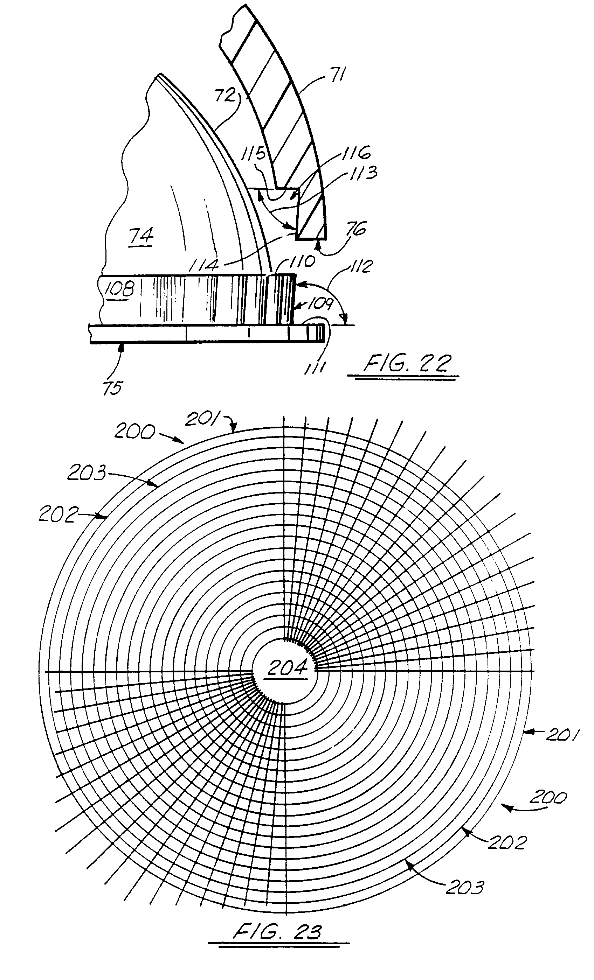

In Figure 22, the connection between cup 71 and liner 72 is illustrated with greater detail. Liner 72 can be rotated as shown by arrows 107 in Figure 16 until the raised portion 75A of base 75 is in a selected position. The liner 70 is then fitted to the cup 71 by engaging the annular recess 116 of the cup 71 with the annular corrugated shoulder 108 of the liner 72. Shoulder 108 has an inclined annular shoulder that forms an acute angle of between about eighty and eighty five degrees with the flat upper surface 111 of annular base 75 of liner 72.

The surface 111 is flat so as to register with the surface of base 76 of cup 71. The numeral 112 in Figure 22 designates the angle between surface 111 and inclined annular wall 109. A recess 116 has a corresponding shape and size to the annular corrugated shoulder 108. The angle 113 formed between inner inclined annular surface 114 and annular surface 115 is the same angular measure as angle 112.

This configuration of annular shoulder 108 and recess 116 provides a snap or interference fit between the liner shoulder 108 and the cup 71 at recess 116 which helps secure the liner 72 to the cup 71 during use.

Figures 23-26 illustrate grid charts 200, 205 that can be drawn on a white sheet of paper for example and used to inspect the highly polished inner concave surface 16 of acetabular cup body 14 for defects. In Figure 23, a grid 200 is in the form of a plurality of concentric rings 201, 202, 203, etc. A central opening 204 allows the user to visually inspect the inside surface 16 of the cup body 14 when the flat grid 200 is placed on top of the cup body 14 with the print of chart 200 facing the mirror like polished concave surface 16. In this fashion, the user simply views the lined pattern of the concentric rings 201-203 of grid chart 200 as reflected off the mirror surface of the inside, concave surface 16 of the cup body 14.

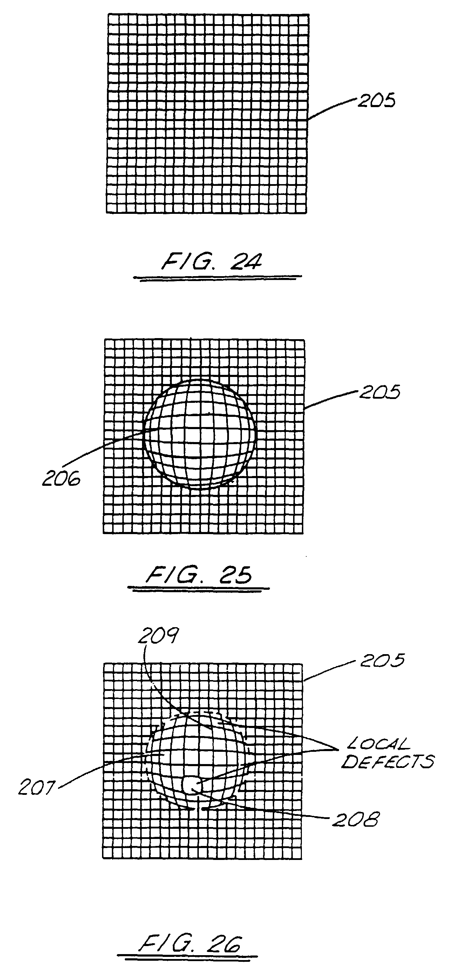

In Figure 24, a generally rectangularly shaped test grid 205 is shown. In Figure 25, a reflective pattern for the test grid 205 is shown as pattern 206, showing no defects. In Figure 26, another test grid pattern reflection 207 is shown that notes two local defects 208, 209. Figure 23 illustrates a method of inspection that may

be by trained human inspectors, simply looking through the central opening 204. When such human inspection is employed, the inspector simply looks through the opening 204 when the grid chart 200, 205 is placed against the concave 16 side of acetabular cup body 14.

Another method of inspection may be by image analysis performed by capturing the reflected image with a video camera, digitizing the image and using computer analysis to measure the amount of deviation of the pattern from the allowed surface geometry tolerance. Thus, the surface 16 defines a mirror that is used as a lens to view a two dimensional pattern such as the grid patterns 200, 205 drawn for example on a white sheet of paper. Distortions in the viewed image are then a result of distortions of the lens surface 16 and hence the surface which is desired to be measured.

The following Table 1 lists part numbers and corresponding par descriptions as used herein and in the drawings:

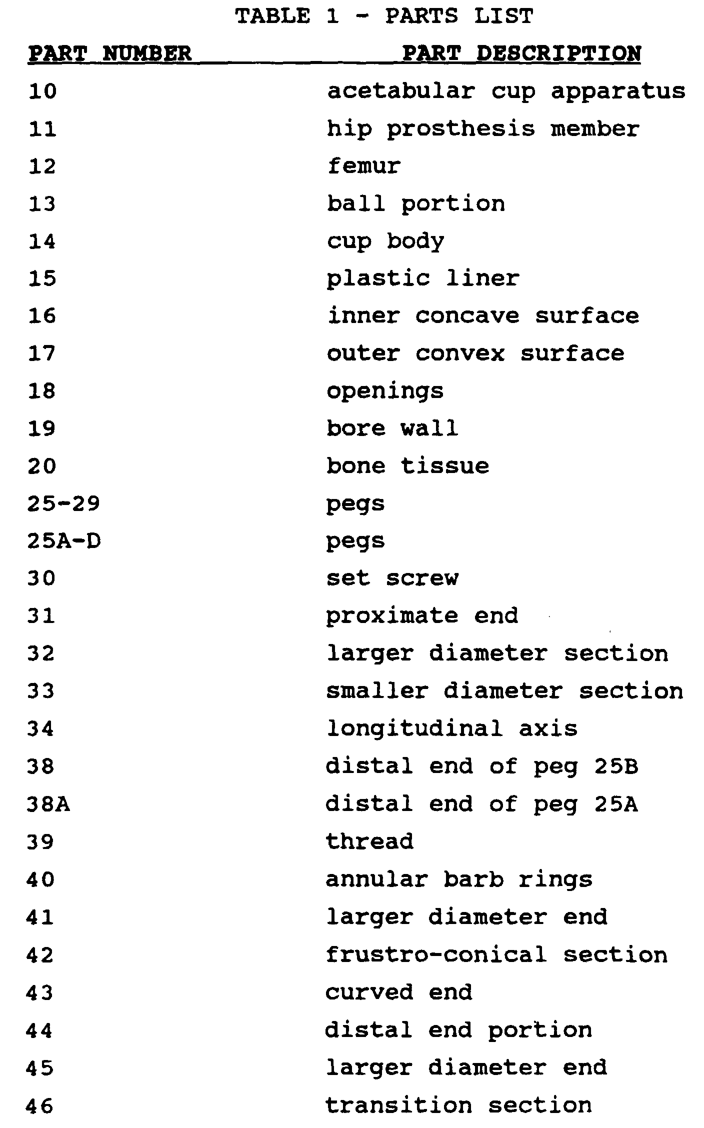

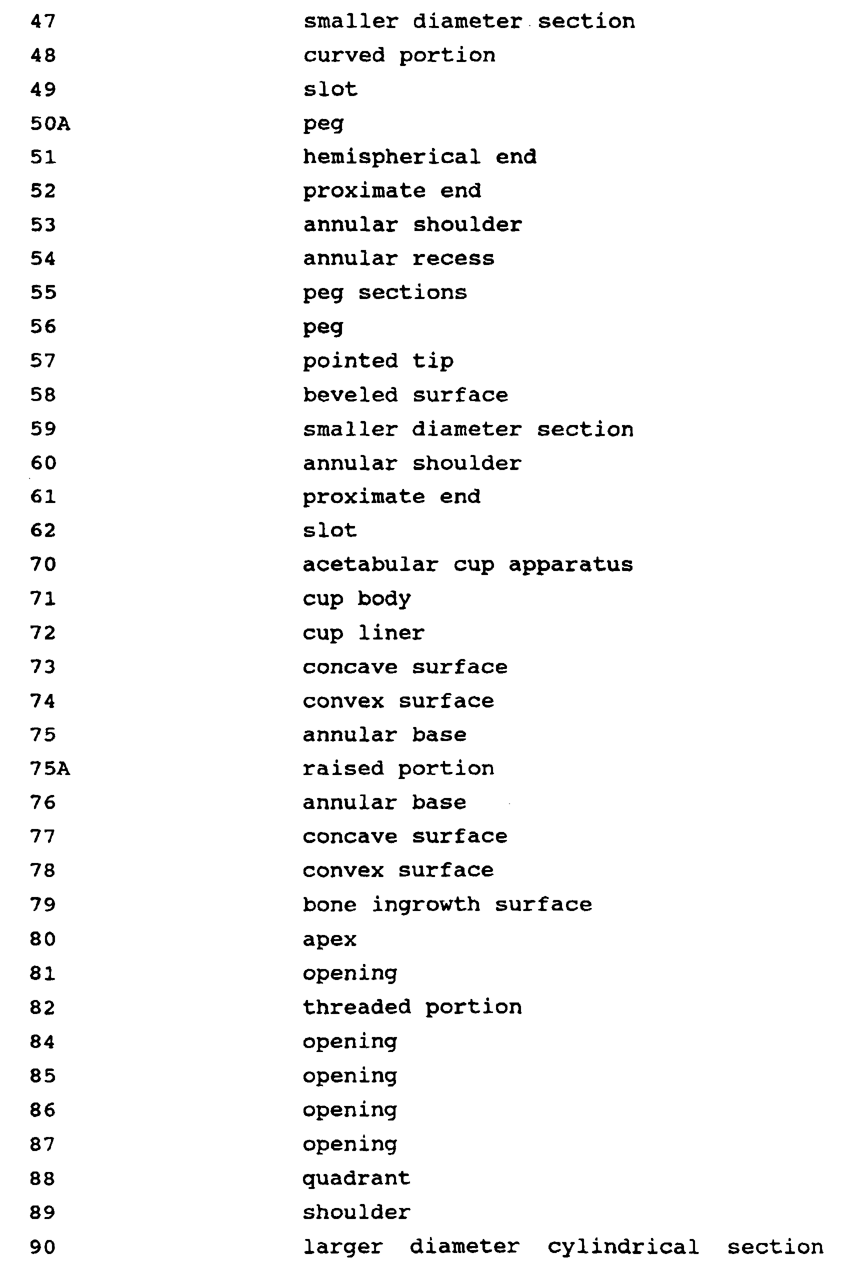

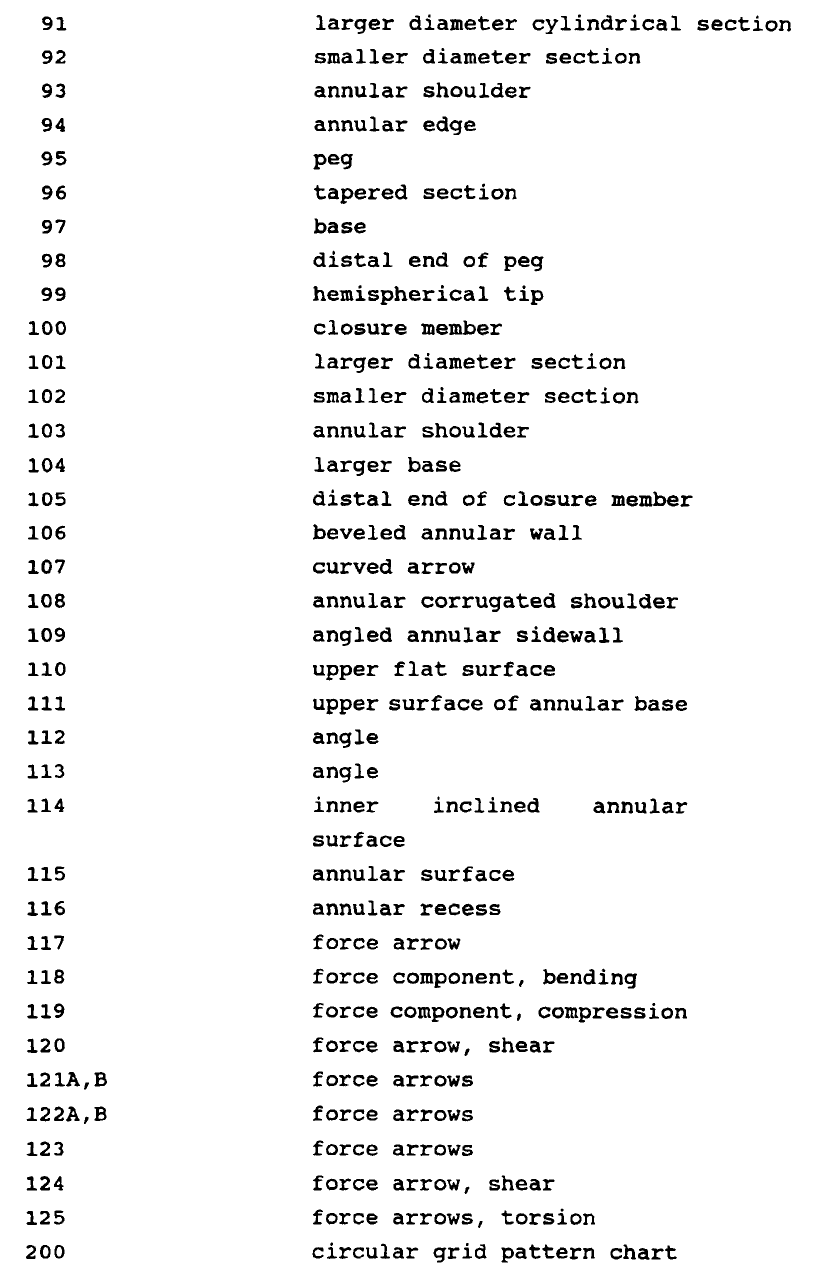

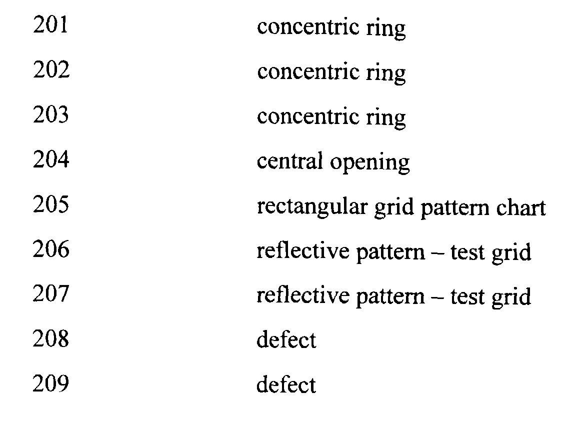

## Claims

1. An arrangement of an acetabular cup prosthesis, comprising:
a) an acetabular cup body (14) having an inner concave surface, an outer convex surface, and an annular base that defines a base plane;
b) a polymeric cup liner (15) that registers with and affixes to the cup body at the cup body concave surface;
c) wherein the cup body concave surface has polished inner surface means that faces the liner for retarding liner debris generation, and including a polished surface having a roughness of less than 20µm (eight micro-inches) characterised in that said arrangement comprises grid means (206, 207) for inspecting the polished inner surface for defects by inspection of the image of the grid viewed in the polished surface.

2. The arrangement of Claim 1, the acetabular cup prosthesis further comprising an array of openings (18) over the cup body and a plurality of peg members (25-29) removably connectable with the cup body at the openings.

3. The arrangement of Claim 2, the acetabular cup prosthesis the openings each defining a smooth walled bore (19).

4. The arrangement of Claim 3 wherein the bore (19) defines a drill guide.

5. The arrangement of Claim 1, wherein the cup body has inner and outer concave (77) and convex (78) surfaces that are of corresponding curvature.

6. The arrangement of Claim 2 wherein each of the openings has a generally cylindrically shaped bore (19) portion.

7. The arrangement of Claim 3 wherein the elongated bores include a generally cylindrical portion and a generally frustro-conical portion.

8. The arrangement of Claim 2, wherein the plurality of peg members (25-29) are each generally cylindrically shaped in configuration.

9. The arrangement of Claim 2, wherein the plurality of peg members (25-29) include a proximal section (32) of larger diameter and a distal section (33) of small diameter.

10. The arrangement of Claim 1, wherein the acetabular cup body is of a metallic material at the bores.

11. An arrangement of an acetabular cup prosthesis, comprising:
a) an acetabular cup body (14) having an inner concave surface and an outer convex surface;
b) a plurality of openings (18) that extend between the inner and outer surfaces along lines that converge substantially near the center of curvature of the inner concave surface, the openings forming elongated bores (19) surrounded by a bore wall portion of the acetabular cup body;
c) one or more peg members (25-29), each being insertable into and registering respectively with one of the openings (18), each peg member having a first proximate end portion having means thereon for forming a substantially rigid connection with the acetabular cup body at one of the openings and with the bore wall that enables load transfer between the cup body and peg members without substantial rotational or translational movement between the cup body and each peg member, and a second distal end portion adapted to extend into the underlying tissue after the cup body has been implanted in a patient; and
d) wherein the cup body concave surface has polished inner surface means that faces the liner for retarding liner debris generation, and including a polished mirror surface characterised in that said arrangement comprises grid means (206, 207) for inspecting the polished inner surface for defects.

12. The arrangement of Claim 11, wherein the polished inner surface means is a mirror-like surface having a roughness of less than 20µm (eight micro inches).

13. The arrangement of Claim 11, wherein the grid means is a grid comprised of a plurality of concentric rings (201-203).

14. The arrangement of Claim 1 or 11 wherein the polymer liner is polyethylene.

15. The arrangement of Claim 11, wherein the mirror surface is adapted to be used as a lens to view the grid means.

16. The arrangement of Claim 1 or 11, wherein the polished inner surface has a roughness of less than 10µm (four micro inches).

17. The arrangement of Claim 1 or 11, wherein the polished inner surface has a roughness of between 2.5 and 10µm (one and four micro inches).

## Patentansprüche

1. Eine Anordnung einer Hüftgelenkpfannen-Prothese mit:
a) einem Hüftgelenkpfannen-Körper (14) der eine innere konkave Oberfläche, eine äußere konvexe Oberfläche und eine ringförmige Basis aufweist, welche eine Basisebene definiert;
b) einer polymeren Pfannenauskleidung (15), welche dem Pfannenkörper angepasst ist und an diesem an dessen konkaver Oberfläche anhaftet;
c) wobei die konkave Oberfläche des Pfannenkörpers eine polierte innere Oberfläche aufweist, die der Auskleidung gegenüberliegt, um die Bildung von Abrieb von der Auskleidung zu hemmen, und eine polierte Oberfläche mit einer Rauhigkeit von weniger als 20 µm (8 Mikro-Inches) vorgesehen ist, **dadurch gekennzeichnet**, dass diese Anordnung eine Gittereinrichtung (206, 207) aufweist, um die polierte innere Oberfläche auf das Vorhandensein von Defekten mittels Untersuchung einer Abbildung des Gitters, welches auf der polierten Oberfläche betrachtet wird, zu untersuchen.

2. Anordnung nach Anspruch 1, bei welcher die Hüftgelenkpfannen-Prothese weiterhin eine Gruppe von Öffnungen (18) über den Pfannenkörper und mehrere Stifte (25-29) aufweist, die lösbar mit dem Pfannenkörper an den Öffnungen verbindbar sind.

3. Anordnung nach Anspruch 2, bei welcher die Öffnungen der Hüftgelenkpfannen-Prothese jeweils eine glattwandige Bohrung (19) definieren.

4. Anordnung nach Anspruch 3, bei welcher die Bohrung (19) eine Bohrer-Führung definiert.

5. Anordnung nach Anspruch 1, bei welcher der Pfannenkörper innere und äußere konkave (77) und konvexe (78) Oberflächen aufweist, die mit einander entsprechenden Wölbungen versehen sind.

6. Anordnung nach Anspruch 2, bei welcher jede Öffnung ein allgemein zylindrisch geformtes Bohrungsteil (19) aufweist.

7. Anordnung nach Anspruch 3, bei welcher die länglichen Bohrungen einen allgemein zylindrischen Teil und einen allgemein kegelstumpfförmigen Teil aufweisen.

8. Anordnung nach Anspruch 2, bei welcher die mehreren Stifte (25-29) jeweils allgemein zylindrisch geformt sind.

9. Anordnung nach Anspruch 2, bei welcher die mehreren Stifte (25-29) einen proximalen Abschnitt (32) mit größerem Durchmesser und einen distalen Abschnitt (33) mit kleinem Durchmesser aufweisen.

10. Anordnung gemäß Anspruch 1, bei welcher der Hüftgelenkpfannen-Körper an den Bohrungen aus metallischem Material besteht.

11. Anordnung einer Hüftgelenkpfannen-Prothese mit
a) einem Hüftgelenkpfannen-Körper (14), der eine innere konkave und eine äußere konvexe Oberfläche aufweist;
b) mehreren Öffnungen (18), die sich zwischen der inneren Oberfläche und der äußeren Oberfläche entlang Linien erstrecken, die im wesentlichen nahe dem Mittelpunkt der Wölbung der inneren konkaven Oberfläche konvergieren, wobei die Öffnungen längliche Bohrungen (19) bilden, die von einem Bohrungswandteil des Hüftgelenkpfannen-Körpers umgeben sind;
c) einem oder mehreren Stiften (25-29), von denen jeder in eine der Öffnungen (18) einführbar und daran angepasst ist, wobei jeder Stift einen ersten proximalen Endabschnitt aufweist, an welchem Mittel zum Bilden einer im wesentlichen starren Verbindung mit dem Hüftgelenkpfannen-Körper an einer der Öffnungen und mit der Bohrungswandung vorhanden sind, die eine Lastübertragung zwischen dem Pfannenkörper und den Stiften ohne eine wesentliche Rotations- oder Translationsbewegung zwischen dem Pfannenkörper und jedem Stift ermöglichen, und ein zweiter distaler Endabschnitt vorhanden ist, der so ausgebildet ist, dass er sich in das darunter befindliche Gewebe erstreckt, nachdem der Pfannenkörper in einem Patienten implantiert worden ist; und
d) die konkave Oberfläche des Pfannenkörpers eine polierte innere Oberfläche aufweist, welche der Auskleidung gegenüberliegt, um die Entstehung von Abrieb von der Auskleidung zu hemmen, und eine polierten Spiegelfläche umfaßt, **dadurch gekennzeichnet**, dass die Anordnung ein Gitter (206, 207) aufweist, um die polierte innere Oberfläche auf Fehler zu untersuchen.

12. Anordnung nach Anspruch 11, bei welcher die polierte innere Oberfläche eine spiegelartige Oberfläche mit einer Rauhigkeit von weniger als 20 µm (8 Mikro-Inches) darstellt.

13. Anordnung nach Anspruch 11, bei welcher das Gitter mehrere konzentrische Ringe (201-203) aufweist.

14. Anordnung nach Anspruch 1 oder 11, bei welcher die Polymerauskleidung aus Polyethylen besteht.

15. Anordnung nach Anspruch 11, bei welcher die Spiegelfläche so ausgebildet ist, dass sie als Linse zur Betrachtung des Gitters verwendbar ist.

16. Anordnung nach Anspruch 1 oder 11, bei welcher die polierte innere Oberfläche eine Rauhigkeit von weniger als 10 µm (vier Mikro-Inches) aufweist.

17. Anordnung nach Anspruch 1 oder 11, bei welcher die polierte innere Oberfläche eine Rauhigkeit zwischen 2,5 und 10 µm (ein und vier Mikro-Inches) aufweist.

## Revendications

1. Agencement d'une prothèse formant cavité cotyloïde, comprenant :
a) un corps formant cavité cotyloïde (14) ayant une surface interne concave, une surface externe convexe et une base annulaire définissant un plan de base ;
b) un élément polymère formant chemise intérieure de cavité (15), qui s'aligne et se fixe sur le corps formant cavité au niveau de la surface concave du corps formant cavité ;
c) dans lequel la surface concave du corps formant cavité présente un moyen poli de surface interne qui fait face à l'élément formant chemise intérieure afin de retarder la formation de débris de ce dernier, et comprenant une surface polie ayant une rugosité inférieure à 20 µm (huit micropouces), caractérisé en ce que ledit agencement comprend des moyens formant quadrillages (206, 207) servant au contrôle de la surface interne polie afin de détecter des défauts, par observation de l'image de la grille vue dans la surface polie.

2. Agencement selon la revendication 1, dans lequel la prothèse formant cavité cotyloïde comprend en outre un ensemble d'ouvertures (18), réparties sur le corps formant cavité, et une pluralité d'éléments formant ergots (25 à 29), pouvant être reliés, de manière détachable, au corps formant cavité au niveau des ouvertures.

3. Agencement selon la revendication 2, dans lequel les ouvertures pratiquées dans la prothèse formant cavité cotyloïde définissent chacune un alésage à paroi lisse (19).

4. Agencement selon la revendication 3, dans lequel l'alésage (19) définit un guidage pour le perçage.

5. Agencement selon la revendication 1, dans lequel le corps formant cavité comporte des surfaces interne concave (77) et externe convexe (78) ayant des courbures correspondantes.

6. Agencement selon la revendication 2, dans lequel chacune des ouvertures présente une partie alésage (19) de forme globalement cylindrique.

7. Agencement selon la revendication 3, dans lequel les alésages oblongs comprennent une partie globalement cylindrique et une partie globalement en forme de cône tronqué.

8. Agencement selon la revendication 2, dans lequel la pluralité d'éléments formant ergots (25 à 29) sont chacun de forme globalement cylindrique.

9. Agencement selon la revendication 2, dans lequel la pluralité d'éléments formant ergots (25 à 29) comprennent une partie proximale (32) de plus grand diamètre et une partie distale (33) de petit diamètre.

10. Agencement selon la revendication 1, dans lequel le corps formant cavité cotyloïde est en matériau métallique au niveau des alésages.

11. Agencement d'une prothèse formant cavité cotyloïde, comprenant :
a) un corps formant cavité cotyloïde (14) ayant une surface interne concave et une surface externe convexe ;
b) une pluralité d'ouvertures (18), qui s'étendent entre les surfaces interne et externe suivant des lignes qui convergent sensiblement à proximité du centre de courbure de la surface interne concave, les ouvertures formant des alésages oblongs (19) entourés par une partie formant paroi d'alésage du corps formant cavité cotyloïde ;
c) un ou plusieurs élément(s) formant ergot(s) (25 à 29), chacun d'eux pouvant être inséré dans l'une des ouvertures (18) et étant en alignement avec celle-ci, chaque élément formant ergot présentant une première partie d'extrémité proximale, comportant des moyens prévus pour assurer une connexion sensiblement rigide avec le corps formant cavité cotyloïde au niveau de l'une des ouvertures et avec la paroi d'alésage, qui permet un transfert des sollicitations entre le corps formant cavité et les éléments formant ergots, sans mouvement sensible de rotation ou de translation entre le corps formant cavité et chaque élément formant ergot, ainsi qu'une seconde partie d'extrémité distale, propre à s'étendre dans les tissus sous-jacents, après que le corps formant cavité a été implanté dans le corps d'un patient ; et
d) dans lequel la surface concave du corps formant cavité présente un moyen poli de surface interne qui fait face à l'élément formant chemise intérieure afin de retarder la formation de débris de ce dernier, et comprenant une surface à poli spéculaire, caractérisé en ce que ledit agencement comprend des moyens formant quadrillages (206, 207) servant à la détection de défauts sur la surface interne polie.

12. Agencement selon la revendication 11, dans lequel la surface interne polie est une surface spéculaire ayant une rugosité inférieure à 20 µm (huit micropouces).

13. Agencement selon la revendication 11, dans lequel les moyens formant quadrillages sont une grille composée d'une pluralité d'anneaux concentriques (201 à 203).

14. Agencement selon la revendication 1 ou 11, dans lequel l'élément polymère formant chemise intérieure est en polyéthylène.

15. Agencement selon la revendication 11, dans lequel la surface spéculaire est propre à être utilisée en tant que lentille pour l'observation du moyen formant quadrillage.

16. Agencement selon la revendication 1 ou 11, dans lequel la surface interne polie a une rugosité inférieure à 10 µm (quatre micropouces).

17. Agencement selon la revendication 1 ou 11, dans lequel la surface interne polie a une rugosité comprise entre 2,5 et 10 µm (un à quatre micropouces).
